# EUROPEAN PATENT SPECIFICATION

(11) **EP 3 384 909 B1**
(45) Date of publication and mention of the grant of the patent: **25.12.2024**
(21) Application number: 16871105.9
(22) Date of filing: 17.03.2016
(51) Int. Cl.: A61K 31/4174, A61K 31/46, A61P 11/14, A61K 9/00

(54) **COMBINATION OF OXYMETAZOLINE AND IPRATROPIUM IN TOPICAL NASAL APPLICATION FOR THE TREATMENT OF A COUGH**
KOMBINATION VON OXYMETAZOLIN UND IPRATROPIUM IN TOPISCHER NASALER ANWENDUNG ZUR BEHANDLUNG VON HUSTEN
COMBINAISON D'OXYMÉTAZOLINE ET D'IPRATROPIUM EN APPLICATION TOPIQUE NASALE POUR LE TRAITEMENT DE LA TOUX

(30) Priority: 02.12.2015 MX 2015016549
(43) Date of publication of application: 10.10.2018
(73) Proprietor: Cortés Borrego, Pablo, 10200 Ciudad de México (MX)
(72) Inventor: Cortés Borrego, Pablo, 10200 Ciudad de México (MX)
(74) Representative: Sach, Greg Robert
(86) International application number: PCT/MX2016/000028
(87) International publication number: WO 2017/095210

(56) References cited:
- WO-A1-2014/158119
- WO-A2-03/024433
- US-A1- 2004 053 902
- P.V. DICPINIGAITIS, ET AL.: "Effect of tiotropium on cough reflex sensitivity in acute viral cough", LUNG, vol. 186, no. 6, 12 September 2008 (2008-09-12), Springer-Verlag, Berlin, DE, pages 369 - 374, XP019657458, ISSN: 1432-1750, DOI: 10.1007/S00408-008-9114-6
- R. ECCLES, ET AL.: "Efficacy and safety of topical combinations of ipratropium and xylometazoline for the treatment of symptoms of runny nose and nasal congestion associated with acute upper respiratory tract infection", AMERICAN JOURNAL OF RHINOLOGY, vol. 21, no. 1, 1 January 2007 (2007-01-01), OceanSide Publications, Providence, RI, US, pages 40 - 45, XP055830752, ISSN: 1050-6586, DOI: 10.2500/ajr.2007.21.2902
- MADISON, J. M. ET AL.: "Pharmacotherapy of chronic cough in adults", EXPERT OPINION ON PHARMACOTHERAPY, vol. 4, no. 7, July 2003 (2003-07-01), pages 1039 - 1048, XP 055388477, ISSN: 1465-6566, DOI: doi:10.1517/14656566.4.7.1039
- SMYRNIOS, N. A. ET AL.: "Chronic cough with a history of excessive sputum production: The spectrum and frequency of causes, key components of the diagnostic evaluation, and outcome of specific therapy", CHEST, vol. 108, no. 4, October 1995 (1995-10-01), pages 991 - 997, XP 055388617, ISSN: 0012-3692
- TURNER, R. B. ET AL.: "Epidemiology, pathogenesis, and treatment of the common cold. Annals of Allergy", ASTHMA & IMMUNOLOGY, vol. 78, no. 6, June 1997 (1997-06-01), pages 531 - 540, XP026957673 , ISSN: 1081-1206
- GHAFOURI, M. A. ET AL.: "Sputum changes associated with the use of ipratropium bromide", CHEST, vol. 86, no. 3, September 1984 (1984-09-01), pages 387 - 393, XP 005466420, ISSN: 0012-3692
- PITKARANTA, A. ET AL.: "Combined intranasal ipratropium bromide and oxymetazoline in experimental rhinovirus infection", AMERICAN JOURNAL OF RHINOLOGY, vol. 12, no. 2, March 1998 (1998-03-01), pages 125 - 129, XP 009510683, ISSN: 1050-6586

## Description

The invention is related with the use of the combination of oximetazoline and ipratropium, administrated via intranasally for cough relief. The cough caused by posterior nasal secretion discharge.

### BACKGROUND

Ipratropium, is a derivative of N-isopropil noratropine, with anticholinergic effects in the upper respiratory tract. The basic patent is US 3505337. For symptomatic treatment of the rhinorrhea associated with perennial rhinitis, a nasal spray (Atrovent^{®}) is available in the market.

Oxymetazoline is an imidazol-simpathetic vasoconstrictor preferably used locally intranasal to unblock nasal mucous membrane. The basic patent of oxymetazoline is DE 1117588 Iliadin Merck (R), metered dose aerosol, drops, pediatric aerosol) is available in the market. There is an aerosol containing Ipratropium and Xilometazoline, a similar molecule, available the market (Otrivin Com^{®}).It has been described a combination of oxymetazoline or a derivative of its salt, with ipratropium or a derivative of its salt for the treatment of symptoms of common cold and/or rhinitis and related symptoms such as nasal congestion, sneezing and hypersecretion (WO 2014158119 A1) which is not actually in the market, with the following composition:

| **Ingredients** | **Quantity** |
|---|---|
| Ipratropium Bromide | 0.005% - 0.5%weight/Volume(0.05mg/ml-5mgs/ml) |
| Oximetazoline hydrochloride | 0.005% - 0.5%weight/Volume(0.05mg/ml-5mgs/ml) |
| EDTA disodium | 0.005% - 0.1% weight/volumen |
| Glycerol | 0% - 30.0% weight/volumen |
| Benzalconium Chlorate | 0% - 0.02 weight/volumen |
| Buffer ( pH adjust) | u.s. |
| Water | u.s. |

There is a previous patent number MX 247216 with International Publication WO 03/024433 A2 which comprises the combination of ipratropium bromide and xylometazoline for symptoms associated with common cold based on nasal congestion, sneezing and hypersecretion, caused by viral infections, allergic or perennial rhinitis.

However, a preparation comprising oxymetazoline salt or derivative, in combination with ipratropium bromide salt or derivative, intranasally used, specifically for relief of cough caused by increased discharge runny nose, towards the retro-nasopharynx, or also called, postnasal drip syndrome has not been described and is not currently available in the market.

The oxymetazoline hydrochloride is a sympathetic agent that shrinks arterioles network within nasal mucosa producing long decongestive and antisecretory effects, therefore it has been used in common cold, to permeate nostrils and decrease rhinorrhea (synonym: catarrh, coryza, nasopharyngitis, cold or flu).

Oxymetazoline dilutions used 0.01 for babies of 6 months to two years of age, 0.25% for children aged 2 to 12 years old and 0.50% ai for children and adults aged 12 years or older.

Ipratropium Bromide is an anticholinergic agent that antagonizes the action of acetylcholine released by nerve endings. Nasal hypersecretion associated to common cold and other upper respiratory tract infections involves neurogenic reflexes with release of acetylcholine at the nasosinusal mucosa level. These reflexes are antagonized by atropine and ipratropium bromide (both are anticholinergic, with anti-acetylcholine effect). This has been proven by studies in which nasal fluid was measured in individuals with naturally acquired common cold ¹⁻³.

At the present time, approaches to the use of oxymetazoline and Ipratropium, are:
- For oxymetazoline hydrochloride, because its permeate effect of nose due to reduce congestion of the nasal mucosal superficial vessels (decongestant effect), with concomitant decrease volume occupied by these tissues (particularly the turbinate erectile tissue), increasing nostrils light, which allows more space for air passage when they are blockade during a common cold, nasopharyngitis or allergic rhinitis. Due to this same vasoconstrictor effect also is decreased the nasal fluid production (since the distended vessels release liquid to nostrils (watery discharge of the common cold)⁴⁻⁶. So that, is used to relieve nasal congestion and excess mucus fluid, not to alleviate cough.
- For ipratropium bromide, it has been traditionally used as a bronchodilator in asthma and chronic obstructive pulmonary disease, being its main use by micronebulizer ⁷⁻⁹ or by oral metered dose inhaled aerosol. More recently it has been experimentally used to reduce nasal secretion¹⁰⁻¹¹.

Madison et al., 2003⁶⁷ describes various drug compounds for use in the treatment of chronic cough. Oxymetazoline is established as a treatment for chronic cough, in combination with antibiotics and antihistamines. Smyrnios et al., 1995⁵⁸ describes the treatment of vasomotor rhinitis with intranasal ipratropium bromide. Sinusitis was treated with a combination of antibiotic and dexbrompheniramine maleate plus d-isoephedrine for at least 3 weeks plus a decongestant nasal spray (oxymetazoline hydrochloride) for 5 days. US 2004/0053902 discloses a composition comprising a nasal descongestan, a corticosteroid and an anticholinergic agent useful in the treatment of upper airway conditions such as rhinitis. More specifically, discloses a composition comprising oxymetazoline HCI (as a nasal decongestant), ipratropium bromide (as anticholinergic agent) and the corticosterioid triamcinolone acetate. Neither of the above documents discloses the use of a combination of ipratropium bromide and oxymetazoline hydrochloride in treating cough related to postnasal drip syndrome.

### SUMMARY

The present invention is set out in the appended set of claims.

Particularly, the present invention relates to a combination of ipratropium bromide and oxymetazoline hydrochloride for use in the relief of cough produced by posterior nasal discharge.

In one preferrable embodiment, the combination is adapted to be administered intranasally.

In another preferrable embodiment, the combination is administered in droplets, microdroplets, aerosol, pressurized aerosol, metered dose pressurized aerosol, spray, microspray, atomization, nasal breeze, or by Respimat^{™} methods, Sterimar^{™} nozzle, or any another method of intranasal fluid diffusion, formulated in powder, solution, suspension, emulsion or gel.

In one more preferrable embodiment, the combination is formulated in the form of an aqueous solution comprising 0.525 mg/ml to 0.1 mg/ml ipratropium bromide and 0.5 mg/ml to 0.1 mg/ml of oxymetazoline hydrochloride.

In one more preferrable embodiment, the combination comprises a stabilizer, wherein the stabilizer can be selected from the group consisting of 50% disodium EDTA (disodium ethylenediaminetetraacetate). In another embodiment, the stabilizer is potassium monobasic phosphate at 0.6804 mg/ml.

In one more preferrable embodiment, the combination also comprises a preservative, wherein the preservative is 0.5% benzalkonium chloride (500 mg/ 100 ml).

In one more preferrable embodiment, the combination also comprises pH stabilizing agents, wherein the pH stabilizing agents are 0.1 N hydrochloric acid and 0.1 N sodium hydroxide, in sufficient quantities to adjust the pH to 4.75.

In one more preferrable embodiment, the combination also comprises humectant and wherein the humectant is glycerin at a concentration of 50% (50 g/ 100 ml).

### BRIEF DESCRIPTION OF THE DRAWINGS

Figure 1 illustrates the administration of the ipratropium bromide and oximetazoline combination intranasally in the way of drops or spray, instilled into both nostrils, with hyperextension of the neck, in order to allow drops go to depth nares by gravity.
Figure 2 illustrates a nasal meatus.
Figures 3 and 4 show the evaluation by the method of 0 to 5 (0 = no cough; 5 = incapacitating cough) using graphics of cough presentation during the day and night according to Dr. Hsu⁶⁵ and previously by Archer⁶⁶.
Figures 5 and 6 show evaluation of the efficacy of the combination of oxymetazoline and ipratropium in nasal topical application for the treatment of cough associated with common cold.

### DETAILED DESCRIPTION OF THE INVENTION

The invention is designed for symptomatic treatment of cough due to post nasal drip syndrome using the combination of ipratropium bromide, oxymetazoline hydrochloride, for use in the relief of cough produced by posterior nasal discharge.

The term "symptomatic treatment of cough" refers to a medical and pharmacological intervention, which serves to reduce or eliminate cough, that is to say, as an antitussive agent without necessarily act on the primary etiology of cough (for example, in viruses or bacterial infections, or in the immunologic disturbance which cause allergy), but on the consequence of it, which very often is, postnasal drip syndrome, which directly causes cough.

The cough to which it refers is that produced mainly during viral rhinopharyngitis (the so-called "flu", "colds" or "common cold"), as well as those produced by bacterial infections such as rhinopharyngitis or bacterial rhinosinusitis, which may lengthen the course of cough

Also the cough to which it refers, is one that tends to get worse predominantly during the nights and mornings. This is because in the dorsal decubitus position, secretion flows more easily, towards the human posterior nasopharyngeal region. Or, when is mobilized in the mornings, the secretion that has accumulated during the night.

The term "posterior drip syndrome" (SGNP) refers to the flow of secretion from the nasal and sinus mucosa, which is directed towards the back of the nose, by its natural drainages (meatus, osteomeatal complexes and the nose itself), which then drains through the posterior nasopharyngeal, oro-pharyngeal and hypopharyngeal walls. It is also referred to as Cough Syndrome due to Rhinosinusal Disease or CSRSD, since they do not always show secretion data to produce cough¹².

The etiology of this syndrom is varied, however it is most frequently due to viral or bacterial infection (rhinopharyngitis, flu, infectious rhinitis, rhinosinusitis, etc.) but it can also be due to allergic reactions, or as a result of exposures to different irritants or physical changes of the environment on the mucosa of the upper respiratory tract.

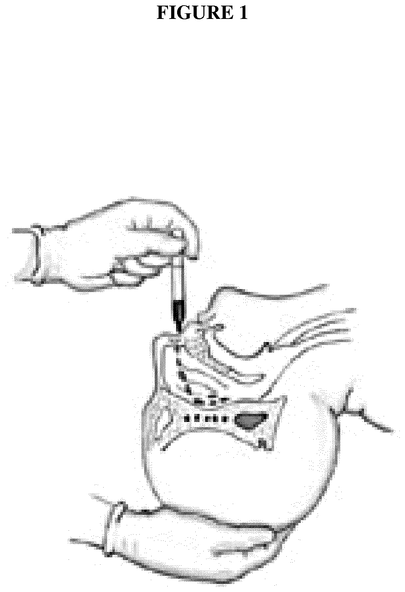

The term "intranasally" refers to the administration of the combination in the way of drops or spray, instilled into both nostrils, with hyperextension of the neck, in order to allow drops go to depth nares by gravity (Figure 1), by allowing a period not less than one minute, then inhale hard, covering the contralateral nostril and then conversely, covering the other nostril to repeat a forced inhalation. The concept is that the solution enters the backest part of the nose. However the combination can be administrated by spray, with head erected, directing the spray up and backward.

The combination may be administered in droplets, microdroplets, aerosol, pressurized aerosol, metered dose pressurized aerosol, spray, microspray, atomization, nasal breeze, or by Respimat^{™} methods. Sterimar^{™} nozzle, or any another method of intranasal fluid diffusion, formulated in powder, solution, suspension, emulsion or gel.

The present invention is the use of a combination of Ipratropium oxymetazoline wherein:

### Quantitative formula

| | |
|---|---|
| Oximetazoline hidrochlorhide | 50.0mg - 25.0 mg - 10.0mg |

| | **Adult - Infant - Pediatric** |
|---|---|
| Ipratropium bromide | 52.5 mg |
| Glycerine | 50.0 g |
| Monobasic potasium fosfate | 68.04 mg |
| Disodium EDTA (disodium etilendiamine-tetra-acetate) | 50.0 mg |
| Benzalconium Chloride | 500.0 mg |
| Sodium hydroxide Solution 0.1 N | To adjust at pH 4.75 |
| Hydrochloric acid 0.1 N | To adjust at pH 4.75 |
| Purified water u.s. | u.s. 100 ml |

u.s. The sufficient to Then, in a preferred embodiment, the combination is formulated in the form of an aqueous solution comprising 0.525 mg/ml to 0.1 mg/ml ipratropium bromide and 0.5 mg/ml to 0.1 mg/ml of oxymetazoline hydrochloride. In another preferred embodiment the combination also comprises a stabilizer that can be selected from the group consisting of 50% disodium EDTA (disodium ethylenediaminetetraacetate), edetic acid, penthenic acid, nitriloacetic acid, malonic acid, pimelic acid, tartaric acid, malic acid, citric acid and its salts. More preferably, the stabilizer is potassium monobasic phosphate at 0.6804 mg/ml. In another preferred embodiment the combination also comprises a preservative, wherein the preservative is 0.5% benzalkonium chloride. (500mg/ 100ml). In another preferred embodiment the combination also comprises pH stabilizing agents, wherein the pH stabilizing agents are 0.1 N hydrochloric acid and 0.1 N sodium hydroxide, in sufficient quantities to adjust the pH to 4.75. In another preferred embodiment the the combination also comprises a humectant and wherein the humectant is glycerin at a concentration of 50% (50 g/ 100ml).

A similar solution, with another different imidazoline derivative than oxymetazoline was used by Dr. Eccles² intranasally to reduce colds nasal obstruction, but not to treat cough. This combination consists of:
Xylometazoline (another derivative of imidazoline similar to oxymetazoline) in different: 1, 0.5, 1.0mg/mL combined with Ipratropium Bromide at a constant dilution 0.6mgs/mL and glycerol, sodium edetate and hydrochloric acid to get a pH 4.5. In a dropper bottle 10mL with each dose of 140µL.

With respect to the previous art in relation to pre-existing cough drugs, literature persistently matches with that current drugs (antihistaminics, decongestants, antitussives, expectorants, bronchodilators, etc.) and other alternative remedies have not been superior than placebo to relief cough, in meta-analysis and reviews of randomized, double-blind, placebo-controlled studies^{13,27}.This includes all over-the-counter drugs (also called self-prescription drugs) such as syrups, expectorants, etc. used to relieve cough of any cause.

So that with respect to the prior art, not an specific antitussive topical nasal are known, directed exclusively towards the most common cause of cough in human being it is, secretion discharge to the back of the nose flow through the oropharynx posterior wall, hypopharynx and larynx with stimulation of nerve endings^{12,28,31}.

Preferred embodiments of the invention are forms: drops, aerosol, pressurized metered dose aerosol, spray or microspray, spray, nasal breeze, nasal spray or by the Respimat^{™}, nozzle Sterimar^{™}, Genuair^{®} devices or any other device to diffuse fluids intranasally.

Physiology of nasal secretions and retronasal flow as a cough producer:
The flow of runny nose consists of several aspects, the glands and goblet cells of the own mucosa of the septum and the wings of the nose, where erectile tissue of the turbinates explain both, congestion and vascular permeation with liquid output into the nasal light. The same process is in fact from the the paranasal sinuses mucous membrane which secretions reaches the nose by draining to nose openings called meatus located, one below the middle turbinate called meatus medium containing other semilunar hiatus (other small openings to nasal light). In the lower hiatus, lead: The frontal sinus, the anterior ethmoid cells and the maxillary sinus, drain at the semilunar hiatus top, where the discharge ends from the rest of the anterior ethmoidal cells. The superior meatus between the superior conchae and the medium turbinate is where secretions flow from the sphenoid sinus and posterior ethmoid cells. Since this meatus is the most posterior, the discharge from it, could flow more easily into the nasopharynx and causing cough.

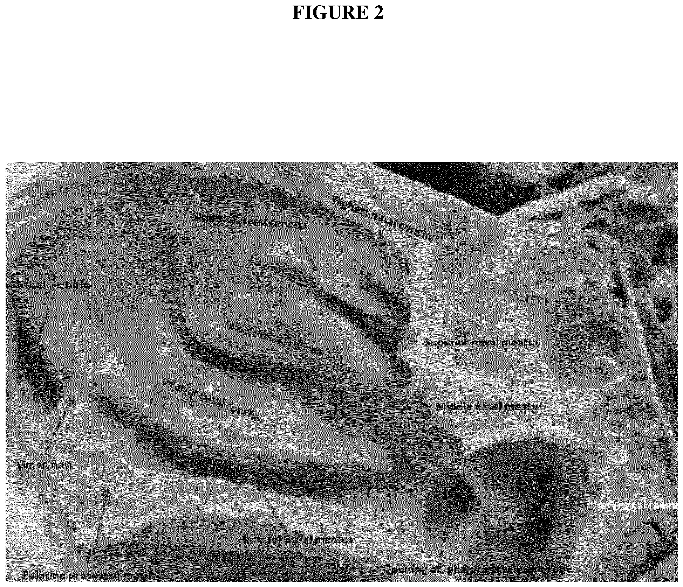

The inferior meatus directly drains in the lacrimal conduit (FIGURE 2).

All this secretions and liquid discharge, does not necessarily go to the front of the nose, as runny nose, but on many occasions it does to the back part of the nose, perhaps according to the meatus which drains and related to the actual position of the individual (supine position facilitates drainage back), causing both, hoarseness and cough.

In children, on the other hand, the ratio between the size of the drainage holes (meatus) and the nasal light is greater than the said ratio (meatus and sinuses) in adults then, some signs and symptoms of rhinosinusitis and colds are different in those age groups because the child produces and eliminates proportionally more secretions mainly through the nose.

Basic parasympathetic nasal physiology, muscarinic and ipratropium bromide: Anticholinergic drugs have utility inter alia, to regulate glandular secretion in allergic rhinitis and sinusitis. The cholinergic reflexes are the most potent in activating exocytosis from submucosal glands respectively in both, lower and upper airways.

The acetylcholine action on postganglionic neurons, regulate specific muscarinic receptors subtypes, located on smooth muscle and submucosa airways glands. 4 receptor types have been defined as M₁, M₂, M₃ and M₄.

Vagal efferent preganglion fibers that produce acetylcholine, originate from tenth dorsal nucleus in brainstem and parasympathetic ganglia innervate larynx and tracheobronchial tree. Innervation of nasal cavity, ethmodial sinuses and posterior parasympathetic, come from preganglion of the seventh pair (facial), originated from the salivary nucleus and passes through the Vidian channel to synapse in sphenopalatine ganglion motor fibers. The postganglionic cholinergic fibers innervate glands in the nasal, pharyngeal and tracheobronchial tree mucosa^{32,33}.

The parasympathetic brainstem nucleus is capable of inducing independent efferent responses in nose, larynx and tracheobronchial tree.

Submucosal glands are densely innervated. Atropine blocks essentially all secretory glands and only partially blocking the blood flow neurogenically induced³⁴⁻³⁵.

Autoradiographic uptake studies indicate that muscarinic receptors are located in submucosal glands, parasympathetic ganglia and nerve branches^{36,37.} In nasal mucosa, M₁ and M₃ receptors are found in glands and in lower levels, in endothelial arterioles cells, in arterio-venous anastomoses, capacitance vessels and venous sinusoids^{37,38}. M₃ receptors predominate in a 2: 1 ratio.

Agonists and antagonists now available do not have sufficient selectivity to discriminate between receptor subtypes. M3 receptors regulate glandular secretion and bronchoconstriction in the human respiratory tract^{38,39}.

M₃ receptors sites and m3mRna gene are distributed through large and small airways, they are found in large concentrations in submucosal glands in which produce exocytosis. M₃ receptors are expressed in lower densities in the nasal epithelium and endothelium

where it is thought, contribute to the goblet cells secretion, ion and water transport, and vasodilatation^{40,41}.

Atropine and ipratropium bromide are antagonists that inhibit cholinergic glandular secretion and bronchoconstriction, mediated by M₃ receptors. Selective antagonists of M₃ , such as Darifenacin (UK-88.525) and Revatropate (UK-112.126), may offer advantages while block receptor during glandular exocytosis without interferon inhibitory mechanisms of M₂ autorreceptors⁴².

*In vivo* cholinergic reflexes act on resistance vessels to increase the blood flow in the nasal surface, but has a limited effect on capacitance vessels (to fill nasal sinusoids leading nasal congestion) controlling mucosal thickness or on venules, that are the site of vascular permeability^{34,43}. M₃ receptors can participate in a limited way in nasal vasodilator reflexes³⁸. Even if small amounts of plasma proteins such as albumin and IgG are released in response to methacholin (parasympathomimetic drug), it does not contribute significantly to the production of secretion. These macromolecules should:
1. - Enter glandular and acinar ducts from the interstitium by diffusion between the cells gap and are expelled along with the large macromolecules in mucus.
2. -Deposited from post-capillary venules, after activation of M₃ receptors in endothelial cells, or
3. -Go out by fenestrated capillaries when the vasodilator effect increases blood pressure transmitted to those previously permeabilized vessels.

Stimulation of nasal nociceptive sensory innervation by cold air, capsaicin, histamine, bradykinin, triggers allergic reactions or parasympathetic reflexes^{10,33,35}. These reflexes are the most important mechanisms in regulate secretion by exocytosis of serous and mucous glands, a finding which has been clearly demonstrated in unilateral nasal provocation models. For example with histamine unilateral challenge, H₁ receptors vessels are stimulated then stimulate vasodilation with sinusoidal filling with blood, leading obstruction of nasal airflow, and increasing permeability with albumin and IgG enriched fluid secretion, it stimulate other tingling sensations that trigger protective reflexes including sneezing. They also triggered bilateral cholinergic reflexes that stimulate glandular secretion of mucoglycoproteins from mucosal cells, lysozyme from serous cells, lactoferin secretory IgA and other non-specific antimicrobial factors. The non cholinergic nasal obstruction mediated reflex component has not been quantified.

Muscarinic antagonist such as atropine and ipratropium bromide effectively reduce glandular secretion and "dry" the mucosa, but have no effect on sneezing or vascular nasal congestion¹⁰. Selective antagonist receptors may have clinical utility in rhinitis, where glandular parasympathetic secretion is an important cause of patient discomfort. However, significant reductions in the volume of glandular secretion can reduce the amounts of antimicrobial and lubricants proteins derived from serous cells on the mucosal surface and may produce dryness, irritation and nerve stimulation.

With respect to the basic physiology sympathetic agonistic action, a receptors and sympathomimetic drugs such as oxymetazoline, venules and veins of nasal mucosa, they are innervated predominantly by sympathetic nerves. However, the arteries that supply the glands are innervated by parasympathetic and adrenergic nerves. Cholinergic stimulation (parasympathetic) dilates arterioles, thereby increasing blood flow to the glands, mucous and serous cells with subsequent secretion of proteins and other substances.

It was demonstrated that α-2 receptors act preferentially on venular ends and α-1 receptors constricts arteries⁴⁴. It is interesting to note that the topical decongestant oxymetazoline, an agonist with high affinity for α-1 and α-2 receptors, produces a dose dependent contraction, with inhibiting effect with α-1 antagonist receptor (prazosin) and minimal inhibitory effect with an α-2 receptor antagonist (yohimbine).

It has been suggested that α-2 agonists predominate in venous sinusoids and congestion regulation⁴⁵. When blood flow decreases through microvasculature and through glands also decrease both, plasma exudation and glandular secretion⁴⁶.

Alpha-adrenoceptors are cell membrane receptors which belong to 7 transmembrane links associated receptor of G protein family. Six genes for α-adrenoceptors have been identified and sequenced⁴⁷.

The α-1 adrenoceptors are coupled via G _{q /11} to C-phospholipase, and receptor activation results in the production of inositol triphosphate (IP3) and diacyl-glycerol, thus an increase in Ca ²⁺ with activation of protein kinases such as Protein Kinase C (PKC).⁴⁸ The α-2 -adrenoceptors are negatively coupled via G i/ ₀ to adenylyl cyclase to decrease cyclic AMP. Activation of α-2 adrenoceptors Q2A causes release inhibition of neurotransmitters. Nasal obstruction associated with acute rhinitis, is probably due to increase of vascular permeability as a result of dilation of the corpus cavernosum and mucosal congestion.

The mucosal of the nasal septum and the lateral tissue of the middle turbinate are intensely vascularized. In the surface of nasal mucosa, blood flows at a rate of 40mL/ 100g tissue per minute⁴⁹.

The mucosal tissue shrinks when exposed to sympathomimetic vasoconstrictor decongestant such as the standard topical oxymetazoline.

The vascular smooth muscle of the nasal mucosa is believed to be the only tissue where has that contractility⁵⁰. Previous studies have shown that α-adrenoceptors are distributed in dogs⁴⁵, pigs⁵¹ and humans^{44,52} in the nasal mucosa and the α-2 adrenoceptors mediate vasoconstriction in the nasal mucosa of pigs and humans. The links-radioligands studies using antagonists to α₁-adrenoceptors (^{[3} H) prazosin and the α₂-adrenoceptor antagonist [3H] rauwolscine, have demonstrated in humans the expression of both proteins adrenoceptorsα₂ - and α₁⁻⁵³. However these radioligands can not discriminate between adrenoceptor subtypes.

In the oxymetazoline and xylometazoline vasoconstriction, the receptor subtypes involved are unknown. That's the same for noradrenaline and adrenaline catecholamines are classified as imidazolines α- adrenoceptor nonselective agonists⁴⁴. Only oxymetazoline had been shown to induce responses Ca²⁺ in heterologous cells expressing human α_{1A}-adrenoceptors⁵⁴.

Controversially, studies indicate that the α-adrenergic stimulation by imidazoles does not decrease, but increase the nasal glandular secretion⁵⁵.

Finally, as a rule, the two catecholamines, norepinephrine and epinephrine, show lower affinities compared to the imidazoles oxymetazoline and xylometazoline⁵⁶. The oxymetazoline can produce a significant increase in blood pressure, fortunately only does it with large doses of 0.3% and no at standard doses of 0.01 to 0.1%⁵⁶. Interactions between sympathetic and parasympathetic systems are not fully known so far, it is known that functionally there is a complex direct interaction between parasympathetic and sympathetic nervous systems, which is of significant importance. This interaction may explain the phenomenon on the other hand has been considered a puzzle.

On one hand sympathetic relationship of noradrenaline released by nerve endings escapes into the blood or into the interstitial fluid, the remainder is inactivated while is received in α-adrenergic polysynaptic effector sites, on target tissues. In fact, when a tissue is exposed to a α-adrenergic-blocker, results in a kind of noradrenaline overflow, which remains active for longer, and may affect other tissues.

The noradrenaline released suggests itself, that its physiological agonist effect exerts an inhibition to release noradrenaline from adrenergic nerve endings, in a inhibitory feedback functional character type, mediated by adrenergic receptors.

Thus, it explains that with various drugs that affect these receptors, different responses are obtained with respect to the release of the noradrenaline neurotransmitter:
α- adrenergic blockers ----------------Increases noradrenaline flow
α-adrenergic agonists ------------------Decreases norepinephrine flow

The preceding shows the role of the direct inhibitory action of the sympathetic nervous system, at least on some of its effectors.⁵⁷ Dually innervated organs (like turbinate nasal mucosa) show a more complex relationship, as sympathetic can inhibit itself by negative feedback. The relationship between sympathetic, through its neurotransmitter noradrenaline and the parasympathetic nerve endings, which are abundant in the capacitance vasculature and nasal submucosal glands, is remarkable.

A research carried out some years ago, studied the effects of sympathomimetic catecholamines in parasympathetic transmission. What was found was a notable capacity of sympathomimetics, noradrenaline and adrenaline, to reduce the acetylcholine (parasympathetic) release from ileum guinea pigs nerve tissue. Noradrenaline reduced acetylcholine discharge in an average 56.8% (20 to 70%) while increasing concentrations of noradrenaline⁵⁸.

Regarding the oxymetazoline effects on parasympathetic system, Starke studied the effect of another sympathetic agent, the oxymetazoline, with relation to the parasympathetic transmission, finding that oxymetazoline significantly inhibited the cardiac response of rabbit vagal electrical stimulation. Parasympathetic transmission was inhibited with decrease in the release of acetylcholine preganglionic and postganglionic nerve endings. In that study, oxymetazoline increased the range of heart beats that had previously decreased during the exposure to acetylcholine. When oxymetazoline infusion was stopped, this effect was rapidly and completely reversed⁵⁹.

Referring to the foregoing, it is apparent that if this happens in other tissues, particularly those with dual innervation, both sympathetic and parasympathetic, as in the nasal mucosa, may oxymetazoline acting on two different vias, when is topically administered:
1. Reducing congestion of the capacitance of the blood vessels due to its adrenergic effect on vascular smooth muscle which in turn could reduce permeability of these vessels that were already increased, for example because of a common cold.
2. Reducing the parasympathetic effect on nasal respiratory mucosa vessels and glands near them.

In relation to imidazoline derivatives, some results have been controversial, an example shows a study in which the xylometazoline decreased rhinorrhea (allegedly decrease in the permeability), even if it did belatedly, till 5 to 10 days of initiate the infectious process (p <0.05)⁶. Unlike the findings of another study, in which no decrease in rhinorrhea was found, on the contrary, increases with the use of xylometazolne when measured on day 1 of administration^{2,49}. An alternative explanation is that these drugs may more active in reducing the permeability, when it has been previously increased by the action of inflammatory substances such as bradykinin⁶⁰, when which are released lately in the course of a viral infection.

Related to this type of infection, also mast cells release histamine locally (perhaps in an earlier phase than the release of bradykinin), which also has an important role in modulating rhinorrhea, possibly via interactions of sympathetic / parasympathetic systems, or acting directly on the mucous glands goblet cells and on blood vessels.

Finally, a knowledge that is relevant to the efficacy of the combination of Oxymetazoline and Ipratropium, is that the affinity of oxymetazoline to α-adrenergic receptors exceeds significantly that affinity for these receptors by noradrenaline^{56.} This increased affinity may cause greater overflow of noradrenaline in tissues, and if this increased overflow (if it is greater) could affect differently, given above, the inhibition of both the sympathetic and parasympathetic fibers in some organs that, as the nose, are doubly innervated.⁶¹ This greater affinity for receptors can result in reduced mucus secretion both, glandular (less parasympathetic effect) and that produced by variations in vascular permeability (less sympathetic effect).

There are some studies where was measured the nasal secretion amount of secretion, not cough, with drugs used. Next, a representative collection of some of them:
Graff³⁰ and Eccles ² mention that using xylometazoline alone, and using ipratropium bromide alone, and the combination of oxymetazoline and ipratropium bromide, found that the combination, after day 1 of treatment, there was a clear separation between nasal symptom scores fluid for placebo and ipratropium in the treated group. The differences were statistically significant compared to placebo (p <0.0001).
- Eccles in 2008⁶, using just xilometazoline: mentioned decreased nasal secretion compared with placebo, statistically significant p <0.05.
- Kim¹¹, Hayden⁶², Diamond⁶³ Dockhorn⁶⁴ in four different studies, using ipratropium alone, intranasally, found lower nasal discharge.

EVALUATION OF THE EFFECTIVENESS OF THE COMBINATION OXYMETAZOLINE AND IPRATROPIUM BROMIDE, INTRANASALY, FOR THE TREATMENT OF COUGH IN ORDER TO ELABORATE THE PATENT REQUEST:
An open pilot study with 6 patients with acute cough associated with common cold, using ipratropium bromide and oxymetazoline combination intranasally (figure 1), administered every 12 hours for 5 days. Patients were 3 to 54 years. Pediatric presentation was used in those of 3 to 12 years hereinafter use the adult presentation. All patients started coughing from 24 to 72 hours before they were enrolled, with clear predominance presenting at night and morning.

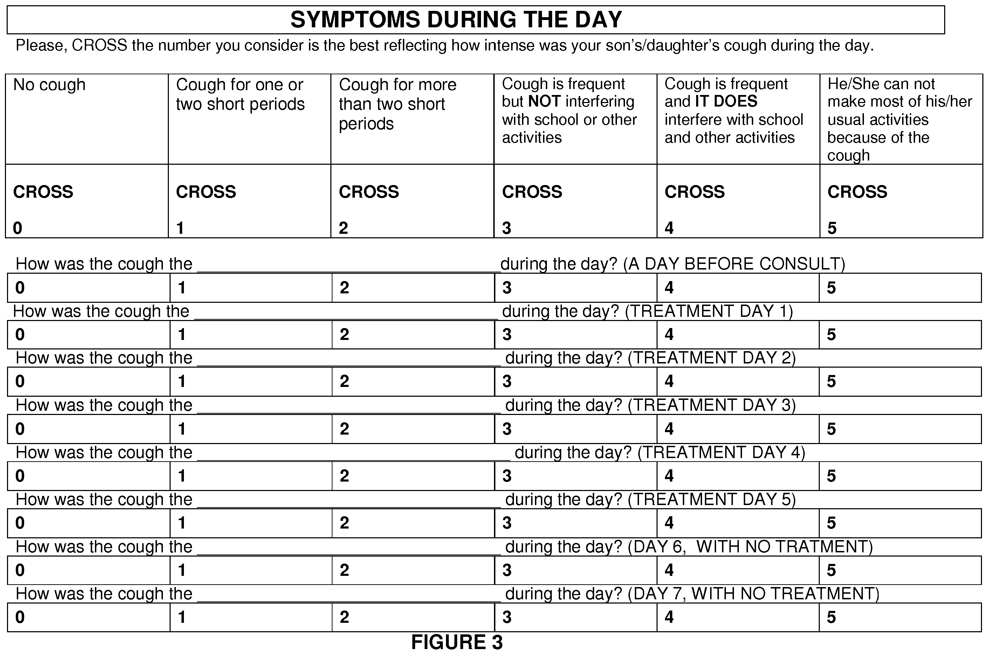

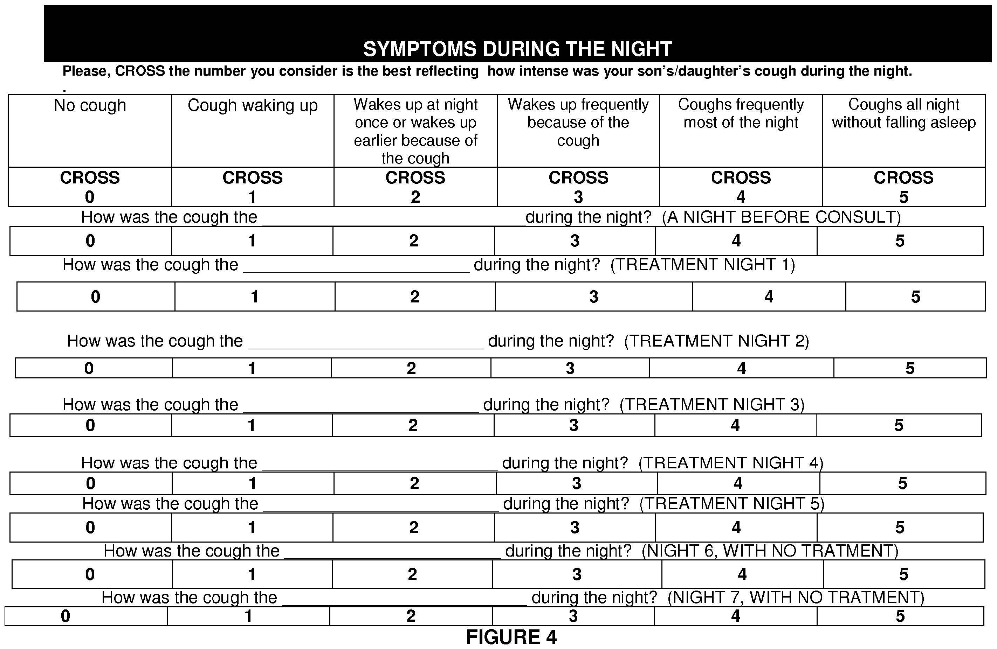

Cough was specifically evaluated by the method of 0 to 5 (0 = no cough; 5 = incapacitating cough) using graphics of cough presentation during the day and night, according to Dr. Hsu⁶⁵ and previously by Archer⁶⁶ (figures 3 and 4). Obtained results showed significant decrease in coughing, both during the day and overnight, with greatest effect on the latter.

During the day, before intranasal administration of the combination drops, from an initial intensity of cough with an average score of 3.0 (frequent cough, which does not interfere with school or other activities), went down to score 1.6 (cough in one or two short periods) the first day and by 1.3 in the second day and finally to an intensity of 0.8 cough (without cough in virtually all patients) on day 5.

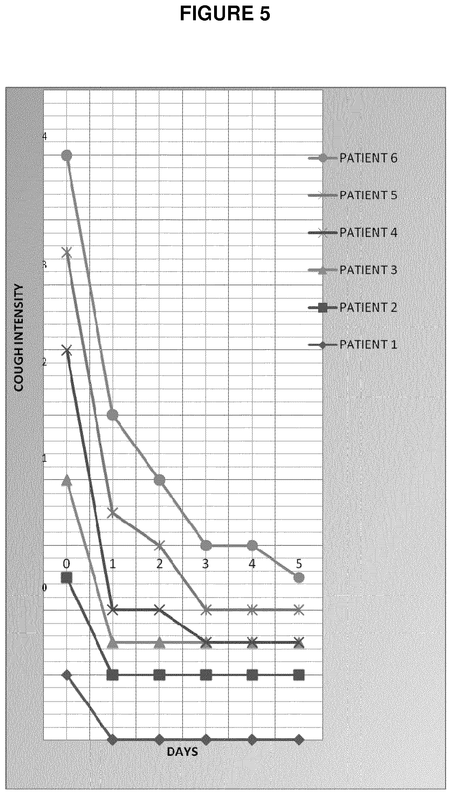

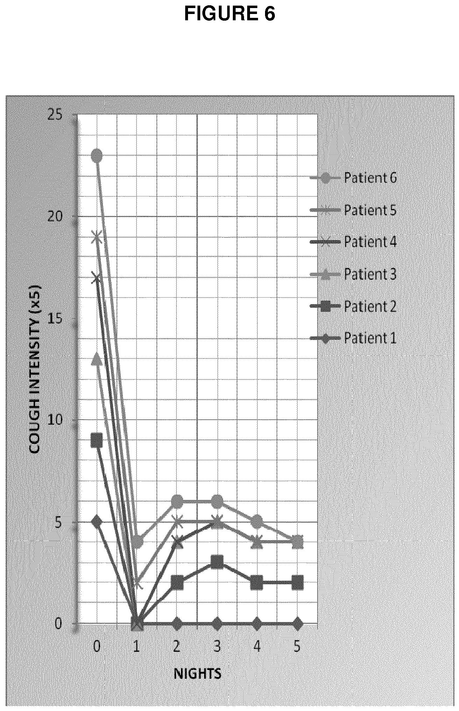

At night, the effect was much more pronounced, starting before treatment with an intensity average cough of 3.8 (frequent cough that in most of patients were most of the night), fell during the combination management in the first night to an average intensity of 0.8, that is, practically disappeared nighttime cough, in a strongest effect showed in this pilot study in all patients evaluated (figures 5 and 6).

At present, the research protocol called "Evaluation of the efficacy of the combination of oxymetazoline and ipratropium in nasal topical application for the treatment of cough associated with common cold" is underway, with a sample of 70 patients of 6 and 6 years. 12 years of age, with a double blind design and controlled by placebo, using the same cough measurement system (Figures 5 and 6).

In summary, the rationale is the following: if in several studies a lower anterior nasal discharge has been demonstrated with the topical use of both drugs, both separately and in combination, it can be assumed that, when said secretion is directed backwards, towards the retronasal region and subsequently retropharyngeal, this decrease leads to a decrease in cough caused by this secretion.

### REFERENCES

1. - Akerlund A, Klint T, Olen L, Rundcrantz H. Nasal Decongestant Effect of Oxymetazoline in the Common Cold: An Objective Dose-Response Study in 106 Patients. J Laryngol Otol 1989: 103 (8): 743-746.
2. - Eccles R, Pedersen A, Regberg D, Tulento H, Borum P, Stjarne P. Efficacy and Safety of Topical Combinations of Ipratropium and Xylometazoline for the Treatment of Symptoms of Runny Nose and Nasal Congestion Associated With Acute Upper Respiratory Tract Infection. Am J Rhinol 2007: 21 (1): 40-45.
3. - Pitkáranta, A, Wecker MT, Korts DC, Hayden FG. Combined Intranasal Ipratropium Bromide and Oxymetazoline in Experimental Rhinovirus Infection. Am J Rhinol. 1998; 12 (2): 125-9. DOI: 10.2500 / 105065898781390316.
4. - Pritchard S, Glover M, Guthrie G, Brum J, Ramsey D, Kappler G, Thomas P, S Stuart, Hull D, Gowland P. Effectiveness of 0.05% Oxymetazoline (Vicks Sinex Micromist®) Nasal Spray in the Treatment of Objective Nasal Congestion Demonstrated to 12 H Post-Administration by Magnetic Resonance Imaging Pulm Pharmacol Ther. 2014 27(1):121-6. Pit: SI 084-5539 (13) 00187-3 doi: 10.1016/j.pupt.2013.08.002.
5. - O'Donell SR, Sympathomimetic vasoconstrictor as nasal descongestants. Med J Aust. 1995 162(5):264-7.
6. - Eccles R, Eriksson M, Garreffa S, Chen SC. The nasal descongestant effect of xiiometazoline in the common cold. Am J Rhinol 15 2008. 22(5), 491-496.
7. - Stokes GM, Milner AD, Hodges IGC, Henry RL. Nebulised ipratropium bromide in wheezy infants and young children. Eur J Respir Dis Suppl. 1983; 128 (Pt 2):494-8.
8. - Stokes GM, Milner AD, Hodges IG, Henry RL, Elphick MC. Nebulised therapy in acute severe bronchiolitis in infancy. Arch Dis Child 1983, 58(5): 279-282.
9. - Moayyedi P, et al Comparison of nebulised salbutamol alone and with ipratropium bromide in the pulmonary treatment of chronic obstructive disease. Thorax 1995; 50 (8):834-7.
10. - Mygind N, Borum P. Anticholinergic treatment of watery rhinorrhea. Am J Rhinol 1990; 4:1-5.
11. - Kim KT, Kerwin E. Landwehr L, Bernstein JA, Bruner D. Harris D, Drda K. Use of 0.06% Ipratropium Bromide Nasal Spray in Children Aged 2 to 5 Years With Rhinorrhea Due to a Common Cold or Allergies. Ann Allergy Asthma Immunol. 2005; 94(1): 73-79.
12. - Pratter MR. Chronic upper airway syndrome secondary to rhinosinus cough diseases (previously referred to as postnasal drip syndrome) ACCP Evidence based clinical practice guidelines. Chest 2006; 129(1 Suppl):63S-71S.
13. - Smith SM, Schroeder K, Fahey T. Over-the-counter medications for acute cough in children and adults in ambulatory settings. Cochrane Database Syst Rev. 2008 Jan 23;(1):CD001831.
14. - Símaserk M, Blandino DA. Treatment of the common Cold. Am Fam Physician 2007; 75(4): 515-20.
15. - Paul IM, Yoder KE, Crowell KR, Shaffer ML, McMillan HS, Carlson LC, Dilworth DA, Berlin CM Jr. Effect of dextromethorphan, diphenhyramine and placebo on nocturnal cough and sleep for coughing quality children and their parents. Pediatrics 2004: 114(1):e85-90.
16. - Brodie M, Graham C, McKean MC. Childhood cough. BMJ 2012; 344: e1177.
17. - Shields MD, Bush A, Everard ML, McKenzie S, Primhak R, BTS Guidelines: Recommendations for the Assessment and Management of Cough in Children. Thorax 2008; 63 (Suppl III)iii1-iii15.
18. - Sung S, Cranswick N. Cough and cold remedies for children. Aust Prescr2009; 32: 122-4.
19. - DeAlleaume L, Tweed B, Neher JO. Do OTC remedies relieve cough in acute URls? J Fam Pract 2009 Oct; S8 (10): 559a-c.
20. - Bell EA, Tunkel DE. Over-the-counter cough and cold medications in children: Are they helpful? Otolarvngol Head Neck Surg. 2010 May; 142 (5): 647-50 doi: 10.1016/j.otohns.2010.01.019.
21. - Shefrin AE, Goldman RD. Use of over-the-counter cough and cold medications in children. Can Fam Physician. 2009 Nov; 55 (11): 1081-3.
22. - Kelley LK, Allen PJ. Managing acute cough in children: evidence-based guidelines. Pediatr Nurs. Nov- Dec 2007: 33 (6): 515-24.
23. - Carr BC. Efficacy, abuse, and toxicity of over-the counter cough and cold medicines in the pediatric population. Curr Opin Pediatr 50, 2006 Apr; 18 (2): 184-8.
24. - Chang AB. Peake J, McEirea MS, Anti-histamines for prolonged non-specific cough in children. Cochrane Database Syst Rev. 2008 apr 16; (2): CD005604, doi: 10.1002/14651858.CD005604.pub3.
25. - Arroll B. No antibiotic treatments for upper-respiratory tract infections (common Cold). Respir Med. 2005 Dec; 99 (12): 1477-1484.
26. -Wang K. Bettiol S, Thompson MJ, Roberts NW, Perera R, Heneghan CJ, Harnden A. Symptomatic treatment of the cough in whooping cough. Cochrane Database Syst Rev. 2014 Sep 22; 9: CD003257. Doi: 10.1002/14651858.CD003257.pub5.
27. - Chang CC, Cheng AC. Chang AB. Over-the-counter (OTC) medications to reduce cough as adjunt to antibiotics for acute pneumonia in children and adults. Cochrane Database Syst Rev. Published Online: 10 MAR 2014 Assessed as up-to-date: 22 JAN 2014 Doi: 10.1002/14851858.CD006088.pub4.
28. - Curley FJ, Irwin RS, Pratter MR, Stivers DH, Doern GV, Vernaglia PA, Larkin AB, Baker SP. Cough in the common cold. Am Rev Resp Dis 1988; 138(5): 305-311.
29. - Committee for the Japanese Respiratory Society Guidelines for Management of Cough. The Japanese Respiratory Society Guidelines for Management of Cough. Respirology 2006 Suppl 4:S135-86).
30. - Graf P, Eccles R. Chen S. Efficacy and safety of intranasal xylometazoline and ipratropium in Patients with common cold. Expert Opin Pharmacother. (2009) 10 (5): 889-908.
31. - Chung KF, Lalloo UG. Diagnosis and management of chronic persistent dry cough. Postgrad Med J 1998; 72(862): 594-598.
32. - Uddman R. Sundler F, Innervation of the upper airways. Clin Chest Med 1986; 7(2): 201.
33. - Baraniuk JN. Neural Control of human nasal secretion. Pulm Pharmacol 1991; 4(1): 20-31.
34. - Lundberg JM, Anggard A, Fahrenkrug J. Complementary role of vasoactive intestinal polypeptide (VIP) and acetylcholine for submandibulargland blood flow and secretion. Acta Physiol Scand 1981: 113(3): 329-36.
35. - Stijarne P. Lacroix JS, Angaard A, Lundberg JM. Compartment analysis of vascular effects of neuropeptides and Capsaicin in the pig nasal mucosa. Acta Physiol Scand 1991: 141(3):335-42.
36. - Barnes PJ, Nadel JA, Roberts JM, Basbaum CB: Muscarinic Receptors in Lung and Trachea: Autoradiographic Localization Using [3H]quinuclidinyl Benzilate. Eur J Pharmacol 1982: 86(1): 103-6.
37. - Okayama M, Baraniuk JN, Merida M, Kaliner MA. Characterization and autoradiographic localization of histamine H1 receptors in human nasal turbinates. J Allergy Clin Immunol 1992; 89: 1144-50.
38. - Baraniuk JN, Kaliner MA, Barnes PJ, Localization of muscarinic M3 receptor mRNA in human nasal mucosa. Am J Rhinol 1992a; 6:145-148.
39. - Eglen RM, Reddy H, Watson N, Challis RAJ. Muscarinic acetylcholine receptor subtypes in smooth muscle. Trends Pharmacol Sci. 1994; 15(4):114-9.
40. - McCormack DG, Mak JC, Minette P, Barnes PJ. Muscarinic receptor subtypes mediating vasodilation in the pulmonary artery. Eur J Pharmacol 1988; 158(3):293-7.
41. - Tokuyama K, Kuo HP, Rohode JAL, Barnes PJ, Rogers OF. Neural Control of goblet cell secretion in guinea pig airways. Am J Physiol 1990; 259(2 Pt 1):L108-15.
42. - Alabaster VA. Discovery and development of selective M3 antagonists for clinical use. Life Sci 1997: 60: 1053-1060.
43. - Raphael GD, Baraniuk JN, Kaliner MA. How and why the nose runs. J Allergy Clin Immunol 1991 87(2):457-67.
44. - Corboz MR. Rivelli MA Varty L, et al. Pharmacological Characterization of Postjunctional α-Adrenoceptors in Human Nasal Mucosa. Am J Rhino I. 2005; 19(5):495-502.
45. - TL Berridge, Roach AG. Characterization of alpha-adrenoreceptors in the vasculature' of canine nasal mucosa. Br Pharmacol 1986; 88(2): 345-354.
46. - O'Donell SR. Sympathomimetic vasoconstrictors as nasal descongestants Med J Aust.1995 162(5):264-7.
47. - Bylund DB, Bond RA Clarke DE Et al. Adrenoceptors. The IUPHAR Compendium of receptor characterization and classification. IUPHAR Media. London UK, 1998:58-74.
48. - Docherty JR. Subtypes of functional alpha1 and alpha2 Eur J Pharm Ichimuramacol adrenoceptors. 1998: 361(1):1-15.
49. - Druce HM, Bonner RF, Patow C, Choo Q, Kaliner MA. Response of nasal blood flow to neurohormones as 50 laser-Doppler Measured by velocimetry. J Appl Phisiol. Respir Environ Exerc Physiol. 1984 57(4): 1276-83.
50. - lchimura K, Jackson RT, Evidence of Alpha 2-adrenoceptors in the Nasal Blood Vessels of the Dog. Arch Otolaryngol. 1984 110(10):647-51.
51. - Corboz MR, Rivelli MA, Varty L, et al. Pharmacological characterization of postjunctional α-adrenoceptors in human nasal mucosa. Am J Rhinol. 2005, 19(5): 495-502.
52. - Anderson KE, Bende M. Adrenoceptors in the Control of human nasal mucous membrane blood flow. Ann Otol Rhinol Laryngol 1984; 1984;93(2 Pt 1):179-82.
53. - Van Megen Y, Aassen AB, Rodriguez de Miranda JF, Van Ginneken CA Wentges BT. Alterations of adrenoceptors in the nasal mucosa of allergic Patients in Comparison with nonalleric individuals. J Allergy Clin Immunol. 1991 87(2):530-40.
54. - Horie K, Obiika K, Foglar R, Tsuimoto G. Selectivity of the imidazoline alpha-adrenoceptor agonists (oxymetazoline and cirazoline) for human cloned alpha 1-adrenoceptor subtypes. Br J Pharmacol. 1995: 116(1): 1611-1618.
55. - Malm L. Vascular and secretory effect of adrenoceptor agonists and peptides in the nose. Eur J Respir Dis Suppl. 1983;128 (Pt 1):139-42.
56. - B Haenisch B, Walstab J, Herberhold S, et al. Alpha-adrenoceptor agonistic activity of oxymetazoline and xylometazoline. Fundam Clin Pharmacol. 2010;24(6):729-739.
57. - Jansson G, Martinson J. Studies on the ganglionic site of action of sympathetic outflow to the Stomach. 1986 Acta Physiol Scand 68(2), 184-192.
58. - Patón WD, Vizi ES. The inhibitory action of noradrenaline and adrenaline on acetylcholine output by guinea-pig ileum longitudinal muscle strip. Br J Pharmacol 1969; 35(1): 10-28.
59. - Starke K. Alpha sympathomimetic inhibition of adrenergic and cholinergic transmission in the rabbit heart. Naunyn Schmiedebergs Arch Pharmacol. 1972;274(1):18-45.
60. - Proud D, Reynolds CJ, Lacapra S, Kagey-Sobolka A, Lichtenstein LM, Naclerio RM. Nasal provocation with bradykinin induces symptoms rhinitis and a sore throat. Am Rev Resp Dis 1988; 137(3):613-6.
61. - Mujic M, Van Rossum JM: Comparative pharmacodynamics of sympathomimetic imidazolines; studies on intestinal smooth muscle of the rabbit and the cardiovascular system of the cat. Arch Int Pharmacodyn Ther. 1965 Jun; 155(2):432-49.
62. - Hayden FG, Diamond L, Wood PB, Korts DC, Wecker MT. Effectiveness and safety of intranasal ipratropium bromide in common colds. A randomized, double blind, placebo controlled trial. Ann Intern Med. 1996 125(2):89-97.
63. - Diamond L, Dockhorn RJ, Kisicki JC, et al. A dose-response study of the safety and efficacy of ipratropium bromide nasal spray in the treatment of the common Cold. J Allergy Clin Immunol 1995; 95 (5 Pt 2) 1139-46.
64. - Dockhorn RJ, Grossman J, Posner M, Zinny M, Tinkleman D. A double blind, placebo controlled study of a safety and efficacy of ipratropium bromide nasal spray versus placebo in patients with common cold. J Allergy Clin Immunol. 1992;90 (6 Pt 2):1076-1082.
65. - Archer LN, Simpson H. Night counts cough and diary-card scores in asthma; Arch Dis Child 1985; 60(5): 473-474.
66. - Hsu JY, Stone RA, Logan-Sinclair RB, Worsdell M, Bussi CM, Chung KF. Coughing in Patients with Frequency persistent cough: assessment using 24 hour ambulatory recorder. Eur Respir J. 1994.7(7), 1246-1253.
67. - Madison JM, Irwin RS. Pharmacotherapy of chronic cough in adults. Expert Opin Pharmacother 2003; 4(7):1039-1048.
68. - Smyrnios NA, Irwin RS. Curley FJ. French CL. From a prospective study off chronic cough. Chest 1995; 108(4): 991-997.

## Claims

1. A combination of ipratropium bromide and oxymetazoline hydrochloride for use in the relief of cough produced by posterior nasal discharge.

2. The combination for use according to claim 1, wherein the combination is adapted to be administered intranasally.

3. The combination for use according to claim 1, wherein the combination is administered in droplets, microdroplets, aerosol, pressurized aerosol, metered dose pressurized aerosol, spray, microspray, atomization, nasal breeze, or by Respimat^{™} methods, Sterimar^{™} nozzle, or any other method of intranasal fluid diffusion, formulated in powder, solution, suspension, emulsion or gel.

4. The combination for use according to claim 1, wherein the combination is formulated in the form of an aqueous solution comprising 0.525 mg/ml to 0.1 mg/ml ipratropium bromide and 0.5 mg/ml to 0.1 mg/ml of oxymetazoline hydrochloride.

5. The combination for use according to claim 4, also comprising a stabilizer, wherein the stabilizer can be selected from the group consisting of 50% disodium EDTA (disodium ethylenediaminetetraacetate).

6. The combination for use, according to claim 4, also comprising a preservative, wherein the preservative is 0.5% benzalkonium chloride (500 mg/100 ml).

7. The combination for use according to claim 4, also comprising pH stabilizing agents, wherein the pH stabilizing agents are 0.1 N hydrochloric acid and 0.1 N sodium hydroxide, in sufficient quantities to adjust the pH to 4.75.

8. The combination for use, according to claim 4, comprising humectant and wherein the humectant is glycerin at a concentration of 50% (50 g/100 ml).

9. The combination for use according to claim 4, also comprising a stabilizer, wherein the stabilizer is potassium monobasic phosphate at 0.6804 mg/ml.

## Patentansprüche

1. Kombination aus Ipratropiumbromid und Oxymetazolinhydrochlorid zur Verwendung bei der Linderung von Husten, der durch hinteren Nasenausfluss verursacht wird.

2. Kombination zur Verwendung nach Anspruch 1, wobei die Kombination für die intranasale Verabreichung geeignet ist.

3. Kombination zur Verwendung nach Anspruch 1, wobei die Kombination in Form von Tröpfchen, Mikrotröpfchen, Aerosol, unter Druck stehendem Aerosol, unter Druck stehendem Aerosol mit Dosierdosis, Spray, Mikrospray, Zerstäubung, Nasenbrise oder durch Respimat^{™}-Methoden, Sterimar^{™}-Düse oder jede andere Methode der intranasalen Flüssigkeitsdiffusion verabreicht wird, die als Pulver, Lösung, Suspension, Emulsion oder Gel formuliert wird.

4. Kombination zur Verwendung nach Anspruch 1, wobei die Kombination in Form einer wässrigen Lösung formuliert ist, die 0,525 mg/ml bis 0,1 mg/ml Ipratropiumbromid und 0,5 mg/ml bis 0,1 mg/ml Oxymetazolinhydrochlorid umfasst.

5. Kombination zur Verwendung nach Anspruch 4, die auch einen Stabilisator umfasst, wobei der Stabilisator aus der Gruppe ausgewählt werden kann, die aus 50% Dinatrium-EDTA (Dinatriumethylendiamintetraacetat) besteht.

6. Kombination zur Verwendung nach Anspruch 4, die auch ein Konservierungsmittel umfasst, wobei das Konservierungsmittel 0,5% Benzalkoniumchlorid (500 mg/100 ml) ist.

7. Kombination zur Verwendung nach Anspruch 4, die auch pH-Stabilisatoren umfasst, wobei die pH-Stabilisatoren 0,1 N Salzsäure und 0,1 N Natriumhydroxid in ausreichenden Mengen sind, um den pH-Wert auf 4,75 einzustellen.

8. Kombination zur Verwendung nach Anspruch 4, die ein Feuchthaltemittel umfasst, wobei das Feuchthaltemittel Glycerin in einer Konzentration von 50% (50 g/100 ml) ist.

9. Kombination zur Verwendung nach Anspruch 4, die auch einen Stabilisator umfasst, wobei der Stabilisator Kaliummonophosphat in einer Konzentration von 0,6804 mg/ml ist.

## Revendications

1. Combinaison de bromure d'ipratropium et de chlorhydrate d'oxymétazoline pour une utilisation dans le soulagement de toux produite par un écoulement nasal postérieur.

2. Combinaison pour une utilisation selon la revendication 1, dans laquelle la combinaison est adaptée pour être administrée par voie intranasale.

3. Combinaison pour une utilisation selon la revendication 1, dans laquelle la combinaison est administrée en gouttelettes, microgouttelettes, aérosol, aérosol sous pression, aérosol sous pression à dose mesurée, pulvérisation, micropulvérisation, atomisation, souffle nasal, ou par des méthodes Respimat^{™}, buse Sterimar^{™}, ou toute autre méthode de diffusion de fluide intranasal, formulée en poudre, solution, suspension, émulsion ou gel.

4. Combinaison pour une utilisation selon la revendication 1, dans laquelle la combinaison est formulée sous la forme d'une solution aqueuse comprenant 0,525 mg/ml à 0,1 mg/ml de bromure d'ipratropium et 0,5 mg/ml à 0,1 mg/ml de chlorhydrate d'oxymétazoline.

5. Combinaison pour une utilisation selon la revendication 4, comprenant également un stabilisant, dans laquelle le stabilisant peut être choisi dans le groupe constitué d'EDTA disodique à 50 % (éthylènediaminetétraacétate disodique).

6. Combinaison pour une utilisation selon la revendication 4, comprenant également un conservateur, dans laquelle le conservateur est du chlorure de benzalkonium à 0,5 % (500 mg/100 ml).

7. Combinaison pour une utilisation selon la revendication 4, comprenant également des agents de stabilisation du pH, dans laquelle les agents de stabilisation du pH sont de l'acide chlorhydrique 0,1 N et de l'hydroxyde de sodium 0,1 N, en quantités suffisantes pour ajuster le pH à 4,75.

8. Combinaison pour une utilisation selon la revendication 4, comprenant un humectant et dans laquelle l'humectant est de la glycérine à une concentration de 50 % (50 g/100 ml).

9. Combinaison pour une utilisation selon la revendication 4, comprenant également un stabilisant, dans laquelle le stabilisant est du phosphate monobasique de potassium à 0,6804 mg/ml.
